(19) Europäisches Patentamt
European Patent Office
Office européen des brevets

(11) EP 0 578 550 B1

(12) **FASCICULE DE BREVET EUROPEEN**

(45) Date de publication et mention de la délivrance du brevet:
**27.12.1996 Bulletin 1996/52**

(51) Int Cl.[6]: **C07C 51/367**, C07C 59/64, C07C 59/52

(21) Numéro de dépôt: **93401731.0**

(22) Date de dépôt: **05.07.1993**

(54) **Procédé de para-hydroxyalkylation de composés aromatiques hydroxylés**

Verfahren zur para-Hydroxyalkylierung von hydroxylierten aromatischen Verbindungen

Process for the para-hydroxyalkylation of hydroxylated aromatic compounds

(84) Etats contractants désignés:
**DE FR GB IT NL SE**

(30) Priorité: **10.07.1992 FR 9208578**

(43) Date de publication de la demande:
**12.01.1994 Bulletin 1994/02**

(73) Titulaire: **RHONE-POULENC CHIMIE**
**92408 Courbevoie Cédex (FR)**

(72) Inventeur: **Nobel, Dominique**
**F-69270 Fontaines-Saint-Martin (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
- **PATENT ABSTRACTS OF JAPAN vol. 12, no. 491 (C-554)(3338) 21 Décembre 1988 & JP-A-63203631**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 578 550 B1

## Description

La présente invention a pour objet un procédé de para-hydroxyalkylation de composés aromatiques hydroxylés.

La présente invention vise un procédé de préparation de composés p-hydroxymandéliques éventuellement substitués et plus particulièrement la préparation de l'acide p-hydroxymandélique et de l'acide méthoxy-3 p-hydroxy-mandélique.

Dans l'exposé qui suit de l'invention, on entend par "composé aromatique hydroxylé", tout composé aromatique porteur d' au moins un groupe hydroxyle et dont la position en para de ce groupe hydroxyle est libre.

On désigne par "composé p-hydroxymandélique éventuellement substitué" un composé aromatique au moins porteur d'un groupe -CHOH-COOH en position para d'un groupe hydroxyle.

L'une des voies de synthèse classique des acides p-hydroxymandéliques consiste à effectuer la condensation en milieu alcalin, de l'acide glyoxylique sur le phénol et/ou ses dérivés correspondants.

Le rendement est limité par le fait que la réaction de condensation n'est pas sélective et conduit également aux acides o-hydroxymandéliques et aux acides dimandéliques.

De plus, le rendement réactionnel est diminué en raison d'une réaction secondaire parasite. En effet, l'acide glyoxylique en milieu alcalin aqueux, est transformé selon la réaction de Cannizaro, en acides oxalique et glycolique.

Pour éviter que cette réaction de Cannizaro devienne prépondérante et détruise l'acide glyoxylique, on a proposé selon FR-A 2 132 364 de conduire la réaction de condensation, en milieu aqueux dilué et à basse température ou température ambiante.

Afin d'améliorer la régio-sélectivité de la réaction, on a proposé selon EP-A 0 368 696 d'effectuer la condensation de l'acide glyoxylique avec le phénol, en présence d'une amine. L'amine utilisée est une amine tertiaire liquide à la température ambiante et insoluble dans l'eau, telle que par exemple, la tributylamine, la trioctylamine, la triisooctylamine ou leurs mélanges. Un des inconvénients dudit procédé est que les rendements ne sont pas très satisfaisants puisque variant de 70 à 74 %. De plus, le rapport molaire phénol/acide glyoxylique est très élevé ce qui nécessite le recyclage d'une quantité importante de phénol. Enfin, une autre contrainte de ce procédé est que la réaction est conduite en milieu biphasique ce qui implique la séparation des phases aqueuse et organique.

Afin d'obvier aux inconvénients précités, la demanderesse propose un nouveau procédé de préparation d'un composé p-hydroxymandélique éventuellement substitué selon un procédé de para-hydroxyalkylation d'un composé aromatique hydroxylé.

La présente invention a précisément pour objet un procédé de parahydroxyalkylation d'un composé aromatique hydroxylé dont la position en para du groupe hydroxyle est libre caractérisé par le fait que l'on effectue la condensation en présence d'un hydroxyde d'ammonium quaternaire:

- d'un composé aromatique hydroxylé de formule générale (I) :

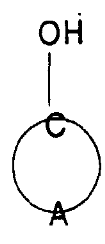

(I)

dans ladite formule (I) :

  - A symbolise un reste carbocyclique aromatique ayant au moins 5 atomes dans le cycle éventuellement substitué, monocyclique ou polycyclique,
  - A porte au moins un groupe hydroxyle et la position en para du groupe hydroxyle est libre,

- et un composé carbonylé répondant à la formule générale (II) :

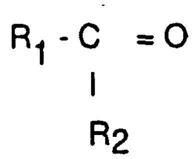

(II)

dans ladite formule (II) :

- $R_1$ représente un atome d'hydrogène, un radical alkyle linéaire ou ramifié ayant de 1 à 6 atomes de carbone ou un radical phényle,
- $R_2$ représente un groupe électro-attracteur.

Conformément au procédé de l'invention, il a été trouvé que la mise en oeuvre d'un hydroxyde d'ammonium quaternaire à la place d'un hydroxyde de métal alcalin permet d'accroître la sélectivité de l'invention.

Un autre avantage du procédé de l'invention est que la réaction peut être conduite en milieu aqueux, en l'absence de tout solvant organique.

Dans l'exposé qui suit de la présente invention, on entend par "groupe électro-attracteur", un groupe tel que défini par H.C. BROWN dans l'ouvrage de Jerry MARCH - Advanced Organic Chemistry, chapitre 9, pages 243 et 244.

A titre d'exemples de groupes électro-attracteurs convenant à la présente invention, on peut citer:

- un groupe -CHO,
- un groupe acyle $R_3$- CO- ou $R_3$- CO $\{CH_2\}_m$- dans lequel $R_3$ représente un radical alkyle linéaire ou ramifié ayant de 1 à 6 atomes de carbone et m représente un nombre de 1 à 3,
- un groupe -$COOR_4$ dans lequel $R_4$ représente un atome d'hydrogène ou un radical alkyle linéaire ou ramifié ayant de 1 à 6 atomes de carbone,
- un groupe -$CX_2H$ dans lequel X représente un atome d'halogène, de préférence, un atome de fluor, chlore ou brome,
- un groupe -$CX_3$ dans lequel X représente un atome d'halogène, de préférence, un atome de fluor, chlore ou brome,

Comme exemples de composés carbonylés répondant à la formule (II), on peut citer:

- le glyoxal,
- l'acide glyoxylique,
- le chloral, le bromal, le fluoral,
- la dichloroacétone,
- la trifluoroacétone,
- la trifluroacétophénone,
- la trifluoroacétylacétone,
- le pyruvate de méthyle, le pyruvate d'éthyle,

Parmi les composés carbonylés précités, l'acide glyoxylique est préféré.

Le procédé de l'invention s'applique tout particulièrement au phénol mais aussi aux phénols substitués ayant au moins une position en para non substituée.

Le noyau aromatique est porteur d'au moins un groupe hydroxyle mais il peut être également porteur d'un ou plusieurs autres substituants. Généralement, par plusieurs substituants, on définit moins de quatre substituants par noyau aromatique.

N'importe quel substituant peut être présent dans la mesure où il n'interfère pas dans la réaction de l'invention.

Ainsi, le procédé de l'invention est bien adapté pour s'appliquer aux composés aromatiques hydroxylés répondant à la formule suivante (I) :

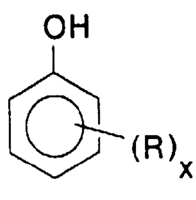
(I)

dans ladite formule (I) :

- la position en para est libre,
- x est un nombre entier compris entre 1 et 4,
- R représente:

- . un atome d'hydrogène,
- . un groupe hydrocarboné ayant de 1 à 20 atomes de carbone choisi parmi les groupes alkyle, alkoxy, hydroxyalkyle, cycloalkyle, aryle, phénoxy, alkoxyalkyle, fluoroalkyle, hydroxyalkoxyalkylène,
- . un groupe hydroxyle,
- . un groupe -CHO,
- . un groupe acyle ayant de 2 à 6 atomes de carbone,
- . un atome d'halogène, de préférence un atome de fluor, de chlore ou de brome.
- . deux groupes R placés sur deux atomes de carbone vicinaux peuvent former ensemble et avec les atomes de carbone qui les portent un cycle benzénique.

On donne ci-après des exemples de radicaux R susceptibles d'être portés par le noyau aromatique:

- radicaux alkyle tels que méthyle, éthyle, propyle, isopropyle, butyle, isobutyle, sec-butyle, tert-butyle, n-octyle, éthyl-2 hexyle, décyle, octadécyle, eicosyle,
- radicaux alkoxy tels que méthoxy, éthoxy, propoxy, isopropoxy, butoxy, hexyloxy, décyloxy, hexadécyloxy, octadécyloxy ou un radical phénoxy,
- radicaux hydroxyalkyle tels que hydroxyméthyle, hydroxyéthyle, hydroxypropyle, hydroxyhexyle, hydroxydécyle,
- radicaux cycloalkyle tels que cyclopentyle, cyclohexyle, cycloheptyle,
- radicaux fluoroalkyle tels que fluorométhyle, difluorométhyle, trifluorométhyle, fluoroéthyle, trifluoro-1,1,1 éthyle, pentafluoroéthyle, fluoropropyle, fluorobutyle, trifluoroamyle,
- radicaux hydroxyalkyoxyalkylène tels que hydroxyméthyloxyéthylène, hydro-xyéthyl di-(oxyéthylène), hydroxyéthyl tri-(oxyéthylène), hydroxyéthyloxy-propylène-1,2, hydroxyéthyloxybutylène, hydroxypropyloxypropylène, hydroxy-butyloxybutylène, hydroxybutyl di-(oxybutylène),
- atomes d'halogène tels que fluor, chlore, brome ou iode.

On met en oeuvre tout préférentiellement dans le procédé de l'invention, les composés aromatiques hydroxylés répondant à la formule générale (I) dans laquelle:

- x est égal à 0, 1, 2 ou 3,
- R représente l'un des groupes ou fonctions suivantes:

  - . un atome d'hydrogène,
  - . un radical alkyle, linéaire ou ramifié, ayant de 1 à 10 atomes de carbone, de préférence de 1 à 4 atomes de carbone,
  - . un radical alkoxy linéaire ou ramifié ayant de 1 à 10 atomes de carbone, de préférence de 1 à 4 atomes de carbone,
  - . un groupe - OH,
  - . un groupe - CHO,
  - . un atome d'halogène.
  - . un groupe - $CF_3$

Encore plus préférentiellement, on choisit les composés de formule (I) dans laquelle les radicaux R identiques ou différents sont un atome d'hydrogène, un radical alkyle, linéaire ou ramifié ayant de 1 à 4 atomes de carbone tels que les radicaux méthyle, éthyle, n-propyle, isopropyle, n-butyle ou isobutyle, un radical alkoxy linéaire ou ramifié ayant de 1 à 4 atomes de carbone comme les radicaux méthoxy ou éthoxy, un groupe - CHO ou un atome de chlore et x est de préférence égal à 0 ou 1.

A titre illustratif de composés répondant à la formule (I), on peut mentionner:

- ceux répondant à la formule (I) dans laquelle x est égal à 0, tels que le phénol,
- ceux répondant à la formule (I) dans laquelle x est égal à 1, tels que:

  - . la pyrocatéchine
  - . la résorcine
  - . l'o-crésol
  - . le m-crésol
  - . l'éthyl-2 phénol
  - . l'éthyl-3 phénol
  - . le propyl-2 phénol

- le sec-butyl-2 phénol
- le tert-butyl-2 phénol
- le tert-butyl-3 phénol
- le méthoxy-2 phénol (gaïacol)
- le méthoxy-3 phénol
- l'éthoxy-2 phénol (guétol)
- l'isopropoxy-2 phénol
- l'aldéhyde salicylique
- le salicylate de méthyle
- le chloro-2 phénol
- le chloro-3 phénol
- le nitro-3 phénol

- ceux répondant à la formule (I) dans laquelle x est égal à 2, tels que:

  - le diméthyl-2,3 phénol
  - le diméthyl-2,5 phénol
  - le diméthyl-3,5 phénol
  - l'hydroxy-2 acétamido-5 benzaldéhyde
  - l'hydroxy-2 éthamido-5 benzaldéhyde
  - le dichloro-2,3 phénol
  - le dichloro-2,5 phénol
  - le dichloro-3,5 phénol
  - le pyrogallol

- ceux répondant à la formule (I) dans laquelle x est égal à 3, tels que:

  - le triméthyl 2,3,5 phénol
  - le di-tert butyl-3,5 phénol
  - le trichloro-2,3,5 phénol

- ceux répondant à la formule (I) présentant un radical naphtalénique, tels que:

  - le naphtol-1
  - le naphtol-2
  - le dihydroxy-1,2 naphtalène
  - le dihydroxy-1,5 naphtalène
  - le dihydroxy-2,3 naphtalène
  - le dihydroxy-2,6 naphtalène
  - le dihydroxy-2,7 naphtalène
  - le bromo-6 naphtol-2

- ceux répondant à la formule (I) présentant un enchaînement de noyaux benzéniques:

  - le phénoxy-2 phénol
  - le phénoxy-3 phénol

Parmi la liste des composés précités, les composés aromatiques porteurs d'au moins un groupe hydroxyle mis en oeuvre préférentiellement sont : le phénol, l'o-crésol, le m-crésol, l'éthyl-3 phénol, le tert-butyl-2 phénol, le gaïacol, le guétol, l'isopropoxy-2 phénol.

Conformément au procédé de l'invention, on effectue la condensation d'un composé aromatique hydroxylé de formule (I) avec un composé carbonylé de formule (II), en présence d'un hydroxyde d'ammonium quaternaire.

Conviennent plus particulièrement à la mise en oeuvre du procédé de l'invention, les hydroxydes d'ammonium quaternaire répondant à la formule (III) suivante:

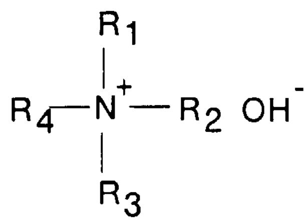

(III)

dans ladite formule (IIII), les radicaux $R_1$, $R_2$, $R_3$ et $R_4$, identiques ou différents représentent:

- un radical alkyle, éventuellement substitué, linéaire ou ramifié, ayant de 1 à 20 atomes de carbone, de préférence de 1 à 12 atomes de carbone,
- un radical aryle, éventuellement substitué, ayant de 6 à 12 atomes de carbone, de préférence de 6 à 10 atomes de carbone,
- un radical aralkyle, éventuellement substitué, ayant de 7 à 14 atomes de carbone, de préférence de 7 à 10 atomes de carbone,
- deux desdits radicaux $R_1$ à $R_4$ pouvant former ensemble un radical alkylène, linéaire ou ramifié ayant de 3 à 6 atomes de carbone.

Dans la formule (III), les radicaux $R_1$, $R_2$, $R_3$ et $R_4$ peuvent représenter un radical alkyle dont la chaîne hydrocarbonée peut être interrompue par un hétéroatome tel que notamment oxygène ou azote et/ou porteuse d'un ou plusieurs groupements ou fonctions tels que notamment, phényle, hydroxyle, halogène, nitro, alkoxy ou alkoxycarbonyle ayant de 1 à 6 atomes de carbone etc....

Dans le cas où les radicaux $R_1$, $R_2$, $R_3$ et $R_4$ comprennent un radical cyclique, notamment benzénique, il est possible que celui-ci soit aussi porteur d'un substituant. A titre exemples de tels substituants, on peut se référer à la liste de groupements et fonctions précités.

Les radicaux $R_1$, $R_2$, $R_3$ et $R_4$ représentent préférentiellement un radical alkyle ayant de 1 à 6 atomes de carbone, de préférence méthyle, éthyle, butyle ; un radical phényle ou un radical benzyle.

Comme exemples d'hydroxyde d'ammonium quaternaire répondant à la formule (III), on met en oeuvre préférentiellement, les hydroxydes de tétralkylammonium ou de trialkylbenzylammonium dont les radicaux alkyle identiques ou différents représentent une chaîne alkyle linéaire ou ramifiée ayant de 1 à 12 atomes de carbone, de préférence de 1 à 6 atomes de carbone.

On choisit préférentiellement l'hydroxyde de tétraméthylammonium, l'hydroxyde de tétraéthylammonium ou l'hydroxyde de tétrabutylammonium.

Il est également possible selon l'invention d'avoir recours à des hydroxydes de trialkylbenzylammonium et notamment à l'hydroxyde de triméthylbenzylammonium.

En ce qui concerne les concentrations et les quantités de réactifs à mettre en oeuvre, on définit ci-après les conditions préférées.

Conformément au procédé de l'invention, on fait appel à une solution d'un composé carbonylé de formule (II). La concentration de ladite solution n'est pas critique et peut varier largement, par exemple, entre 15 et 100 % en poids. On a recours, d'une manière préférée, lors de la mise en oeuvre de l'acide glyoxylique à titre de composé carbonylé de formule (II), aux solutions commerciales d'acide glyoxylique dont la concentration est d'environ 50 %.

Selon le procédé de l'invention, on fait réagir le composé carbonylé de formule (II) sur le composé aromatique hydroxylé de formule (I). Le rapport molaire entre le composé aromatique hydroxylé de formule (I) et le composé carbonylé de formule (II) varie entre 0,3 et 4,0 et est choisi préférentiellement entre 0,5 et 2,0.

La solution d'hydroxyde d'ammonium quaternaire mise en oeuvre a une concentration généralement comprise entre 10 et 50 % en poids. La concentration de la solution de départ n'est pas critique. Toutefois, comme la concentration du composé aromatique hydroxylé de formule (I) est avantageusement faible dans le milieu réactionnel, on utilise une solution diluée de l'hydroxyde d'ammonium quaternaire pour effectuer la dilution du milieu réactionnel.

La concentration du composé aromatique hydroxylé de formule (I) est comprise de préférence entre 0,5 et 1,5 moles/litre, et plus particulièrement aux environs de 1 mole/litre.

La quantité d'hydroxyde d'ammonium quaternaire introduite dans le milieu réactionnel tient compte de la quantité nécessaire pour salifier tous les groupements salifiables du composé aromatique hydroxylé de formule (I) et du composé carbonylé de formule (II) qui peuvent être des groupes hydroxyle et/ou des fonctions carboxylique COOH.

La quantité d'hydroxyde d'ammonium quaternaire peut varier largement et être égale ou voisine de la stoechiométrie ou en excès. Généralement, la quantité d'hydroxyde d'ammonium quaternaire varie entre 80 et 120 % de la quantité stoechiométrique.

La température de la réaction est choisie avantageusement entre 20°C et 60°C, et de préférence entre 30°C et 40°C.

Le procédé de l'invention est conduit à pression atmosphérique mais sous atmosphère contrôlée de gaz inertes, de préférence d'azote ou de gaz rares, en particulier d'azote.

On donne ci-après un mode préféré de réalisation pratique de l'invention.

Dans un milieu réactionnel comprenant le composé aromatique hydroxylé de formule (I), de l'eau et de l'hydroxyde d'ammonium quaternaire en quantité nécessaire pour salifier le groupe hydroxyle et d'autres éventuelles fonctions salifiables, on introduit la solution du composé carbonylé de formule (II) et en parallèle la solution d'hydroxyde d'ammonium quaternaire mis en oeuvre en une quantité nécessaire pour salifier les éventuelles fonctions salifiables.

Si le composé aromatique hydroxylé de formule (I) et le composé carbonylé de formule (II) présente des fonctions salifiables, on introduit donc également de l'hydroxyde d'ammonium quaternaire en quantité nécessaire pour salifier toutes les fonctions salifiables.

On maintient le milieu réactionnel sous agitation et à la température choisie dans l'intervalle précité pendant une durée variable allant de 1 à 10 heures.

En fin de réaction, on sépare le composé hydroxylé et para-hydroxyalkylé obtenu sous forme salifiée selon les méthodes conventionnelles.

Dans le cas notamment de l'acide p-hydroxymandélique ou de l'acide méthoxy-3 p-hydroxymandélique, ils sont obtenus sous forme salifiée et peuvent être séparés selon les techniques classiques de séparation, notamment par cristallisation.

Le procédé de la présente invention conduit à l'obtention de composés aromatiques hydroxylés et p-hydroxyalkylés.

En particulier, dans sa variante préférée, le procédé de l'invention qui consiste à faire réagir un composé aromatique hydroxylé répondant à la formule (I) avec l'acide glyoxylique permet d'obtenir des composés p-hydroxymandéliques éventuellement substitués qui peuvent être représentés par la formule (IV) suivante :

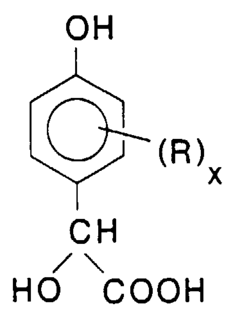

(IV)

dans ladite formule (IV), R et x ayant la signification donnée dans la formule (I).

Ces produits sont particulièrement intéressants car ce sont des produits intermédiaires permettant entre autres, d'obtenir par réduction, des acides hydroxyarylacétiques ou par oxydation, des acides hydroxyarylglyoxyliques (= hydroxyaryl α-oxo acétiques) ou des aldéhydes hydroxyaromatiques.

Une application préférée de l'invention est la préparation d'aldéhydes hydroxyaromatiques, par oxydation des composés de formule (IV) obtenus selon l'invention.

L'oxydation des composés de formule (IV) peut être conduite selon les techniques décrites dans la littérature. Ainsi, on peut se référer à P. HEBERT [Bull. Soc. Chim. France, 27, p.45-55(1920)] et à NAGAI SHIGEKI et al, [JP-A 76/128934]. L'oxydation est généralement conduite par l'oxygène ou l'air sous pression, en présence d'un catalyseur approprié tel que par exemple, les dérivés du chrome, cobalt, cuivre, vanadium ou osmium.

Ainsi, l'invention permet d'accéder facilement à l'hydroxy-4 benzaldéhyde et à la vanilline et ses analogues, par exemple éthyl-3, isopropyl-3 vanilline, par oxydation respectivement de l'acide p-hydroxymandélique et des acides méthoxy-3 p-hydroxymandélique, éthoxy-3 p-hydroxy-mandélique, ou isopropoxy-3 p-hydroxymandélique.

Les exemples qui suivent illustrent l'invention sans toutefois la limiter.

Dans les exemples, les pourcentages indiqués sont exprimés en poids.

Les abréviations mentionnées dans les exemples ont la signification suivante :

$$\text{Conversion (TT)} = \frac{\text{nombre de moles de gaïacol transformées}}{\text{nombre de moles de gaïacol introduites}}$$

$$\text{Sélectivité (RT)} = \frac{\text{nombre de moles d'acide mandélique formées}}{\text{nombre de moles de gaïacol transformées}}$$

Exemple 1

Dans un réacteur en verre d'un litre muni d'une double-enveloppe, d'une électrode de pH, d'une sonde de température, d'un réfrigérant, d'une arrivée de gaz inerte et d'une agitation mécanique, on charge :

- 310 g d'eau distillée,
- 138 g (0,281 mol) d'une solution aqueuse d'hydroxyde de tétraéthylammonium quaternaire à 30 %,
- 69,7 g (0,56 mol) de gaïacol

On établit l'atmosphère inerte et l'on porte le mélange réactionnel à 35°C et l'on ajoute simultanément en 2 heures, 138 g (0,281 mol) de ladite solution aqueuse d'hydroxyde d'ammonium quaternaire à 30 % et 41,6 g d'une solution aqueuse d'acide glyoxylique à 50 %.

On maintient le mélange réactionnel à 35°C pendant 2 heures.

En fin de réaction, on dose les produits de la réaction par chromatographie liquide haute performance.

Les résultats obtenus sont les suivants:

- conversion:
  - . TT = 44,0 %
- acide hydroxy-4 méthoxy-3 mandélique :
  - . RT = 97 %
- acide hydroxy-2 méthoxy-3 mandélique :
  - . RT = 2,7 %
- acide hydroxy-2 méthoxy-3 dimandélique-1,5 :
  - . RT = 1,1 %.

Exemple comparatif 2

On reproduit l'exemple 1 à la différence que l'on charge de la soude à la place de l'hydroxyde de tétraéthylammonium.

Les résultats obtenus sont les suivants :

- conversion :
  - . TT = 43 %
- acide hydroxy-4 méthoxy-3 mandélique :
  - . RT = 88 %
- acide hydroxy-2 méthoxy-3 mandélique:
  - . RT = 4,5 %
- acide hydroxy-2 méthoxy-3 dimandélique-1,5:
  - . RT = 6,9 %

Exemples comparatifs 3 et 4

Exemples 5 à 8

Dans les exemples suivants, on reproduit l'exemple 1 à la différence près que tous les réactifs sont chargés simultanément et que l'on maintient le milieu réactionnel à 35°C, pendant 3 heures.

Les exemples 3 et 4 sont des essais comparatifs où l'hydroxyde d'alkylammonium est remplacé par la soude ou l'ammoniaque.

Les résultats obtenus sont consignés dans le tableau suivant:

Tableau I

| Ref. ex. | hydroxyde d'ammonium quaternaire | TT | RT | | |
|---|---|---|---|---|---|
| | | | para | ortho | di |
| 3 | soude (comparatif) | 42,0 | 60,0 | 4,3 | 2,2 |
| 4 | ammoniaque (comparatif) | 51,0 | 0 | 0 | 2,5 |
| 5 | hydroxyde de tétraméthylammonium | 38,5 | 96,0 | 2,7 | 1,0 |
| 6 | hydroxyde de tétrapropylammonium | 40 | 95,6 | 3,3 | 1,1 |
| 7 | hydroxyde de tétrabutylammonium | 32,0 | 63,5 | 2,0 | 0,7 |
| 8 | hydroxyde de triméthylbenzylammonium | 33,0 | 95,0 | 3,9 | 1,0 |

Dans ledit tableau, les abréviations ortho, para et di signifient:

- acide hydroxy-4 méthoxy-3 mandélique = para
- acide hydroxy-2 méthoxy-3 mandélique = ortho
- acide hydroxy-2 méthoxy-3 dimandélique-1,5 = di

Il ressort de l'examen du tableau (I) que la mise en oeuvre d'un hydroxyde d'ammonium quaternaire permet d'améliorer la sélectivité de la réaction.

## Revendications

**1.** Procédé de para-hydroxyalkylation d'un composé aromatique hydroxylé dont la position en para du groupe hydroxyle est libre caractérisé par le fait que l'on effectue la condensation, en présence d'un hydroxyde d'ammonium quaternaire:

- d'un composé aromatique hydroxylé de formule générale (I) :

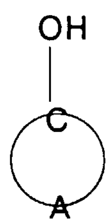

(I)

dans ladite formule (I) :

- A symbolise un reste carbocyclique aromatique ayant au moins 5 atomes dans le cycle éventuellement substitué, monocyclique ou polycyclique,
- A porte au moins un groupe hydroxyle et la position en para du groupe hydroxyle est libre,

- et un composé carbonylé répondant à la formule générale (II) :

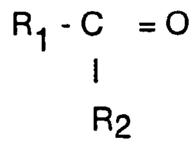

(II)

dans ladite formule (II) :

- $R_1$ représente un atome d'hydrogène, un radical alkyle linéaire ou ramifié ayant de 1 à 6 atomes de carbone ou un radical phényle,
- $R_2$ représente un groupe électro-attracteur.

**2.** Procédé selon la revendication 1 caractérisé par le fait que le composé aromatique hydroxylé répond à la formule suivante (I) :

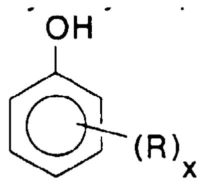

(I)

dans ladite formule (I) :

- la position en para est libre,
- x est un nombre entier compris entre 1 et 4,
- R représente:
  - . un atome d'hydrogène,
  - . un groupe hydrocarboné ayant de 1 à 20 atomes de carbone choisi parmi les groupes alkyle, alkoxy, hydroxyalkyle, cycloalkyle, aryle, phénoxy, alkoxyalkyle,fluoroalkyle, hydroxyalkoxyalkylène,
  - . un groupe hydroxyle,
  - . un groupe -CHO,
  - . un groupe acyle ayant de 2 à 6 atomes de carbone,
  - . un atome d'halogène, de préférence un atome de fluor, de chlore ou de brome.
  - . deux groupes R placés sur deux atomes de carbone vicinaux peuvent former ensemble et avec les atomes de carbone qui les portent un cycle benzénique.

**3.** Procédé selon l'une des revendications 1 et 2 caractérisé par le fait que le composé aromatique hydroxylé répond à la formule (I) dans laquelle :

- x est égal à 0, 1, 2 ou 3,
- R représente l'un des groupes ou fonctions suivantes:
  - . un atome d'hydrogène,
  - . un radical alkyle, linéaire ou ramifié, ayant de 1 à 10 atomes de carbone, de préférence de 1 à 4 atomes de carbone,
  - . un radical alkoxy linéaire ou ramifié ayant de 1 à 10 atomes de carbone, de préférence de 1 à 4 atomes de carbone,
  - . un groupe - OH,
  - . un groupe - CHO,
  - . un atome d'halogène.
  - . un groupe - $CF_3$

**4.** Procédé selon l'une des revendeications 1 à 3 caractérisé par le fait que le composé aromatique hydroxylé répond

à la formule (I) dans laquelle les radicaux R identiques ou différents sont un atome d'hydrogène, un radical alkyle, linéaire ou ramifié ayant de 1 à 4 atomes de carbone, un radical alkoxy linéaire ou ramifié ayant de 1 à 4 atomes de carbone, un groupe - CHO, un atome de chlore et x est de préférence égal à 0 ou 1.

**5.** Procédé selon l'une des revendications 1 à 4 caractérisé par le fait que le composé aromatique hydroxylé de formule (I) est le phénol, l'o-crésol, le m-crésol, l'éthyl-3 phénol, le tert-butyl-2 phénol, le gaïacol, le guétol, l'isopropoxy-2 phénol.

**6.** Procédé selon l'une des revendications 1 à 5 caractérisé par le fait que le composé carbonylé répondant à la formule générale (II) dans laquelle $R_2$ représente un groupe électro-attracteur choisi parmi:

- un groupe -CHO,
- un groupe acyle $R_3$-CO- ou $R_3$-CO$-(CH_2)_m$- dans lequel $R_3$ représente un radical alkyle linéaire ou ramifié ayant de 1 à 6 atomes de carbone et m représente un nombre de 1 à 3,
- un groupe -$COOR_4$ dans lequel $R_4$ représente un atome d'hydrogène ou un radical alkyle linéaire ou ramifié ayant de 1 à 6 atomes de carbone,
- un groupe -$CX_2H$ dans lequel X représente un atome d'halogène, de préférence, un atome de fluor, chlore ou brome,
- un groupe -$CX_3$ dans lequel X représente un atome d'halogène, de préférence, un atome de fluor, chlore ou brome,

**7.** Procédé selon l'une des revendications 1 à 6 caractérisé par le fait que le composé carbonylé répondant à la formule générale (II) est choisi parmi:

- le glyoxal,
- l'acide glyoxylique,
- le chloral, le bromal, le fluoral,
- la dichloroacétone,
- la trifluoroacétone,
- la trifluroacétophénone,
- la trifluoroacétylacétone,
- le pyruvate de méthyle, le pyruvate d'éthyle.

**8.** Procédé selon l'une des revendications 1 à 7 caractérisé par le fait que l'hydroxyde d'ammonium quaternaire mis en oeuvre répond à la formule (III):

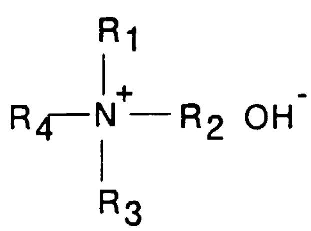

(III)

dans ladite formule (IIII), les radicaux $R_1$, $R_2$, $R_3$ et $R_4$, identiques ou différents représentent:

- un radical alkyle, éventuellement substitué, linéaire ou ramifié, ayant de 1 à 20 atomes de carbone, de préférence de 1 à 12 atomes de carbone, de préférence méthyle, éthyle, butyle,
- un radical aryle, éventuellement substitué, ayant de 6 à 12 atomes de carbone, de préférence de 6 à 10 atomes de carbone, de préférence phényle,
- un radical aralkyle, éventuellement substitué, ayant de 7 à 14 atomes de carbone, de préférence de 7 à 10 atomes de carbone, de préférence benzyle,
- deux desdits radicaux $R_1$ à $R_4$ pouvant former ensemble un radical alkylène, linéaire ou ramifié ayant de 3 à 6 atomes de carbone.

**9.** Procédé selon l'une des revendications 1 à 8 caractérisé par le fait que l'hydroxyde d'ammonium quaternaire mis en oeuvre est un hydroxyde de tétralkylammonium ou un hydroxyde de trialkylbenylammonium dont les radicaux

alkyle identiques ou différents représentent une chaîne alkyle linéaire ou ramifiée ayant de 1 à 12 atomes de carbone, de préférence de 1 à 6 atomes de carbone

**10.** Procédé selon l'une des revendications 1 à 9 caractérisé par le fait que l'hydroxyde d'ammonium quaternaire mis en oeuvre est l'hydroxyde de tétraméthylammonium, l'hydroxyde de tétraéthylammonium, l'hydroxyde de tétrabutylammonium, l'hydroxyde de triméthylbenzylammonium.

**11.** Procédé selon l'une des revendications 1 à 10 caractérisé par le fait que la solution aqueuse du composé carbonylé de formule (II) a une concentration variant de 15 à 100 % en poids.

**12.** Procédé selon l'une des revendications 1 à 11 caractérisé par le fait que le rapport molaire entre le composé aromatique hydroxylé de formule (I) et le composé carbonylé de formule (II) varie entre 0,3 et 4,0 et est choisi préférentiellement entre 0,5 et 2,0.

**13.** Procédé selon l'une des revendications 1 à 12 caractérisé par le fait que la solution aqueuse d'hydroxyde d'ammonium quaternaire a une concentration variant de 10 à 50 % en poids.

**14.** Procédé selon l'une des revendications 1 à 13 caractérisé par le fait que la quantité d'hydroxyde d'ammonium quaternaire varie entre 80 et 120 % de la quantité stoechiométrique nécessaire pour salifier tous les groupements salifiables du composé aromatique hydroxylé de formule (I) et du composé carbonylé de formule (II).

**15.** Procédé selon l'une des revendications 1 à 14 caractérisé par le fait que la concentration du composé aromatique hydroxylé de formule (I) est comprise de préférence entre 0,5 et 1,5 moles/litre, et plus particulièrement aux environs de 1 mole/litre.

**16.** Procédé selon l'une des revendications 1 à 15 caractérisé par le fait que la température de la réaction varie entre 20°C et 60°C, de préférence entre 30°C et 40°C.

**17.** Procédé selon la revendication 1 caractérisé par le fait que l'on effectue la préparation de l'acide méthoxy-3 p-hydroxymandélique par condensation du gaïacol et de l'acide glyoxylique, dans l'eau et en présence d'hydroxyde d'ammonium quaternaire.

**18.** Utilisation du produit obtenu selon le procédé de préparation décrit dans l'une des revendications 1 à 17 comme intermédiaire de fabrication des acides hydroxyarylacétiques, des acides hydroxyarylglyoxyliques ou des aldéhydes hydroxyaromatiques.

**19.** Utilisation de l'acide p-hydroxymandélique et des acides méthoxy-3 p-hydroxymandélique, éthoxy-3 p-hydroxymandélique ou isopropoxy-3 p-hydroxymandélique obtenu selon le procédé de préparation décrit dans l'une des revendications 1 à 17 pour la fabrication de l'hydroxy-4 benzaldéhyde et de la vanilline et analogues par oxydation desdits acides.

**Patentansprüche**

**1.** Verfahren zur p-Hydroxyalkylierung einer hydroxylierten aromatischen Verbindung, deren para-Position zur Hydroxylgruppe frei ist, dadurch gekennzeichnet, daß man in Gegenwart eines quaternären Ammoniumhydroxids eine Kondensation durchführt zwischen:

- einer hydroxylierten aromatischen Verbindung der allgemeinen Formel (I)

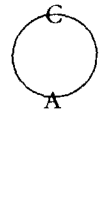

(I)

wobei in dieser Formel (I)

- A einen aromatischen carbocyclischen Rest mit mindestens 5 Atomen im monocyclischen oder polycyclischen, gegebenenfalls substituierten Ring bezeichnet ;
- A mindestens eine Hydroxylgruppe enthält und die para-Position zur Hydroxylgruppe frei ist;

- und einer Carbonylverbindung mit der allgemeinen Formel (II)

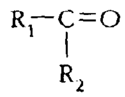

(II)

wobei in dieser Formel (II)

- $R_1$ ein Wasserstoffatom, einen linearen oder verzweigten Alkylrest mit 1 bis 6 Kohlenstoffatomen oder einen Phenylrest darstellt;
- $R_2$ eine elektronenziehende Gruppen bezeichnet.

**2.** Verfahren nach Anspruch 1, dadurch gekennzeichnet, daß die hydroxylierte aromatische Verbindung der Formel (I) entspricht

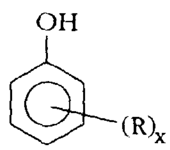

(I)

wobei in dieser Formel (I)

- die para-Position frei ist;
- x eine ganze Zahl zwischen 1 und 4 darstellt;
- R darstellt:
    - ein Wasserstoffatom;
    - eine Kohlenwasserstoffgruppe mit 1 bis 20 Kohlenstoffatomen, ausgewählt aus den Gruppen Alkyl, Alkoxy, Hydroxyalkyl, Cycloalkyl, Aryl, Phenoxy, Alkoxyalkyl, Fluoralkyl und Hydroxyalkoxyalkylen;
    - eine Hydroxylgruppe;
    - eine CHO-Gruppe;
    - eine Acylgruppe mit 2 bis 6 Kohlenstoffatomen;
    - ein Halogenatom, vorzugsweise ein Fluor-, Chlor- oder Bromatom;
    - zwei Gruppen R, befindlich an zwei benachbarten Kohlenstoffatomen, die gemeinsam und mit den Kohlenstoffatomen, die sie tragen, einen Benzolring bilden können.

**3.** Verfahren nach einem der Ansprüche 1 und 2, dadurch gekennzeichnet, daß die hydroxylierte aromatische Verbindung der Formel (I) entspricht, in der

- x gleich 0, 1, 2 oder 3 ist;
- R eine der folgenden Gruppen oder Funktionen darstellt:
    - ein Wasserstoffatom;
    - einen linearen oder verzweigten Alkylrest mit 1 bis 10 Kohlenstoffatomen, vorzugsweise 1 bis 4 Kohlenstoffatomen;
    - einen linearen oder verzweigten Alkoxyrest mit 1 bis 10 Kohlenstoffatomen, vorzugsweise 1 bis 4 Kohlenstoffatomen;
    - eine OH-Gruppe;
    - eine CHO-Gruppe;

- ein Halogenatom;
- eine $CF_3$-Gruppe

**4.** Verfahren nach einem der Ansprüche 1 bis 3, dadurch gekennzeichnet, daß die hydroxylierte aromatische Verbindung der Formel (I) entspricht, in der die Reste R, identisch oder verschieden, ein Wasserstoff, ein linearer oder verzweigter Alkylrest mit 1 bis 4 Kohlenstoffatomen, ein linearer oder verzweigter Alkoxyrest mit 1 bis 4 Kohlenstoffatomen, eine CHO-Gruppe oder ein Chloratom sind und x vorzugsweise gleich 0 oder 1 ist.

**5.** Verfahren nach einem der Ansprüche 1 bis 4, dadurch gekennzeichnet, daß die hydroxylierte aromatische Verbindung der Formel (I) Phenol, o-Kresol, m-Kresol, 3-Ethylphenol, 2-tert.-Butylphenol, Guajakol, Guetol oder 2-Isopropoxyphenol ist.

**6.** Verfahren nach einem der Ansprüche bis 5, dadurch gekennzeichnet, daß die Carbonylverbindung der allgemeinen Formel (II) entspricht, in der $R_2$ eine elektronenziehende Gruppe darstellt, ausgewählt aus:

- einer CHO-Gruppe;
- einer Acylgruppe $R_3$-CO- oder $R_3$-CO$\{CH_2\}_m$-, in der $R_3$ einen linearen oder verzweigten Alkylrest mit 1 bis 6 Kohlenstoffatomen darstellt und m eine Zahl zwischen 1 und 3 darstellt;
- einer Gruppe -$COOR_4$, in der $R_4$ ein Wasserstoffatom oder einen linearen oder verzweigten Alkylrest mit 1 bis 6 Kohlenstoffatomen darstellt;
- eine Gruppe -$CX_2H$, in der X ein Halogenatom, vorzugsweise ein Fluor-, Chlor- oder Bromatom, darstellt;
- eine Gruppe -$CX_3$, in der X ein Halogenatom, vorzugsweise ein Fluor-, Chlor- oder Bromatom, darstellt.

**7.** Verfahren nach einem der Ansprüche 1 bis 6, dadurch gekennzeichnet, daß die Carbonylverbindung, die der allgemeinen Formel (II) entspricht, ausgewählt ist aus:

- Glyoxal;
- Glyoxalsäure;
- Chloral, Bromal, Fluoral;
- Dichloraceton;
- Trifluoraceton;
- Trifluoracetophenon;
- Trifluoracetylaceton;
- Methylpyruvat, Ethylpyruvat.

**8.** Verfahren nach einem der Ansprüche 1 bis 7, dadurch gekennzeichnet, daß das eingesetzte quaternäre Ammoniumhydroxid der Formel (III) entspricht

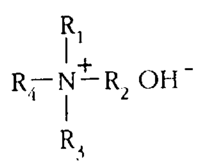 (III)

wobei in der Formel (III) die Reste $R_1$, $R_2$, $R_3$ und $R_4$, identisch oder verschieden, jeweils darstellen:

- einen linearen oder verzweigten, gegebenenfalls substituierten Alkylrest mit 1 bis 20 Kohlenstoffatomen, vorzugsweise mit 1 bis 12 Kohlenstoffatomen, vorzugsweise einen Methyl-, Ethyl- oder Butylrest;
- einen gegebenenfalls substituierten Arylrest mit 6 bis 12 Kohlenstoffatomen, vorzugsweise mit 6 bis 10 Kohlenstoffatomen, vorzugsweise einen Phenylrest;
- einen gegebenenfalls substituierten Aralkylrest mit 7 bis 14 Kohlenstoffatomen, vorzugsweise mit 7 bis 10 Kohlenstoffatomen, vorzugsweise einen Benzylrest;
- zwei dieser Reste $R_1$ bis $R_4$ zusammen einen linearen oder verzweigten Alkylenrest mit 3 bis 6 Kohlenstoffatomen bilden können.

**9.** Verfahren nach einem der Ansprüche 1 bis 8, dadurch gekennzeichnet, daß das eingesetzte quaternäre Ammoniumhydroxid ein Tetralkylammoniumhydroxid oder ein Trialkylbenzylammoniumhydroxid ist, dessen Alkylreste,

identisch oder verschieden, eine lineare oder verzweigte Alkylkette mit 1 bis 12 Kohlenstoffatomen, vorzugsweise 1 bis 6 Kohlenstoffatomen, darstellen.

**10.** Verfahren nach einem der Ansprüche 1 bis 9, dadurch gekennzeichnet, daß das eingesetzte quaternäre Ammoniumhydroxid Tetramethylammoniumhydroxid, Tetraethylammoniumhydroxid, Tetrabutylammoniumhydroxid oder Trimethylbenzylammoniumhydroxid.

**11.** Verfahren nach einem der Ansprüche 1 bis 10, dadurch gekennzeichnet, daß die wäßrige Lösung der Carbonylverbindung der Formel (II) eine Konzentration von 15 bis 100 Gew.-% besitzt.

**12.** Verfahren nach einem der Ansprüche 1 bis 11, dadurch gekennzeichnet, daß das Molverhältnis zwischen der aromatischen Verbindung (I) und der Carbonylverbindung der Formel (II) zwischen 0,3 und 4,0 variiert und vorzugsweise zwischen 0,5 und 2,0 ausgewählt ist.

**13.** Verfahren nach einem der Ansprüche 1 bis 12, dadurch gekennzeichnet, daß die wäßrige quaternäre Ammoniumhydroxidlösung eine Konzentration zwischen 10 und 50 Gew.-% hat.

**14.** Verfahren nach einem der Ansprüche 1 bis 13, dadurch gekennzeichnet, daß die Menge des quaternären Ammoniumhydroxids zwischen 80 und 120 % der stöchiometrischen Menge variiert, die erforderlich ist, um alle versalzbaren Gruppen der hydroxylierten aromatischen Verbindung der Formel (I) und der Carbonylverbindung der Formel (II) zu versalzen.

**15.** Verfahren nach einem der Ansprüche 1 bis 14, dadurch gekennzeichnet, daß die Konzentration der hydroxylierten aromatischen Verbindung der Formel (I) vorzugsweise zwischen 0,5 und 1,5 mol/l, insbesondere etwa 1 mol/l, beträgt.

**16.** Verfahren nach einem der Ansprüche 1 bis 15, dadurch gekennzeichnet, daß die Reaktionstemperatur zwischen 20 °C und 60 °C, vorzugsweise zwischen 30 °C und 40 °C, variiert.

**17.** Verfahren nach Anspruch 1, dadurch gekennzeichnet, daß man die Synthese der 3-Methoxy-p-hydroxymandelsäure durch Kondensation des Guajakols und der Glyoxalsäure in Wasser und in Gegenwart eines quaternären Ammoniumhydroxids durchführt.

**18.** Verwendung des Produkts, erhalten nach dem in einem der Ansprüche 1 bis 17 beschriebenen Syntheseverfahren als Zwischenprodukt zur Herstellung von Hydroxyarylessigsäuren, Hydroxyarylglyoxalsäuren und hydroxyaromatischen Aldehyden.

**19.** Verwendung der p-Hydroxymandelsäure und der 3-Methoxy-p-hydroxymandelsäure, 3-Ethoxy-p-hydroxymandelsäure oder 3-Isopropoxy-p-hydroxymandelsäure, erhalten nach dem in einem der Ansprüche 1 bis 17 beschriebenen Syntheseverfahren zur Herstellung des 4-Hydroxybenzaldehyds und von Vanillin und analogen Verbindungen durch Oxidation dieser Säuren.

## Claims

**1.** A process for the para-hydroxyalkylation of a hydroxylated aromatic compound, whereof the para position of the hydroxyl group is free, characterised in that condensation takes place in the presence of a quaternary ammonium hydroxide:

- of a hydroxylated aromatic compound of general formula (I):

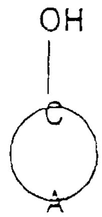

(I)

in which formula:

- A symbolises an aromatic carbocyclic residue having at least 5 atoms in the optionally substituted, monocyclic or polycyclic cycle,
- A carries at least one hydroxyl group and the para-position of the hydroxyl group is free,

- and a carbonyl compound in accordance with the general formula (II):

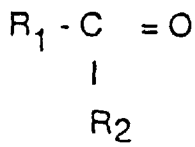

(II)

in which formula (II):

- $R_1$ represents a hydrogen atom, a straight or branched alkyl radical having 1 to 6 carbon atoms or a phenyl radical,
- $R_2$ represents an electro-attracting group.

**2.** A process according to Claim 1, characterised in that the hydroxylated aromatic compound corresponds to the following formula (I):

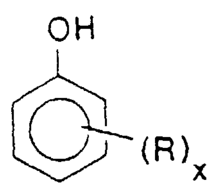

(I)

in which formula (I):

- the para-position is free,
- x is an integer between 1 and 4,
- R represents:
  - . a hydrogen atom,
  - . a hydrocarbon group with 1 to 20 carbon atoms selected from the alkyl, alkoxy, hydroxyalkyl, cycloalkyl, aryl, phenoxy, alkoxyalkyl, fluoroalkyl and hydroxyalkoxyalkylene groups,
  - . a hydroxyl group,
  - . a -CHO group,
  - . an acyl group having 2 to 6 carbon atoms,
  - . a halogen atom, preferably a fluorine, chlorine or bromine atom,
  - . two R groups placed on two neighbouring carbon atoms can form together and with the carbon atoms carrying them a benzene cycle.

**3.** A process according to one of Claims 1 and 2, characterised in that the hydroxylated aromatic compound corresponds to formula (I) wherein:

- x is equal to 0, 1, 2 or 3,
- R represents one of the following groups or functions:
  - . a hydrogen atom,
  - . a straight or branched alkyl radical with 1 to 10 carbon atoms, preferably 1 to 4 carbon atoms,
  - . straight or branched alkoxy radical with 1 to 10 carbon atoms, preferably 1 to 4 carbon atoms,
  - . a -OH group,
  - . a -CHO group,
  - . a halogen atom.
  - . a - $CF_3$ group

**4.** A process according to one of Claims 1 to 3, characterised in that the hydroxylated aromatic compound corresponds to formula (I) wherein the R radicals which are the same or different are a hydrogen atom, a straight or branched alkyl radical with 1 to 4 carbon atoms, a straight or branched alkoxy radical with 1 to 4 carbon atoms, a - CHO group, a chlorine atom, and x is preferably equal to 0 or 1.

**5.** A process according to one of Claims 1 to 4, characterised in that the hydroxylated aromatic compound of formula (I) is phenol, o-cresol, m-cresol, 3-ethyl phenol, 2-tert-butyl phenol, guaiacol, guaiethol, 2-isopropoxy phenol.

**6.** A process according to one of Claims 1 to 5, characterised in that the carbonyl compound corresponding to general formula (II) wherein $R_2$ represents an electro-attracting group chosen from:

- a -CHO group,
- a $R_3$ -CO- or $R_3$-CO $(-CH_2)-_m$- acyl group, in which $R_3$ represents a straight or branched alkyl radical with 1 to 6 carbon atoms, and m represents a number from 1 to 3,
- a $-COOR_4$ group, in which $R_4$ represents a hydrogen atom or a straight or branched alkyl radical with 1 to 6 carbon atoms,
- a $-CX_2H$ group, in which X represents a halogen atom, preferably a fluorine, chlorine or bromium atom,
- a $-CX_3$ group, in which X represents a halogen atom, preferably a fluorine, chlorine or bromine atom.

**7.** A process according to one of Claims 1 to 6, characterised in that the carbonyl compound corresponding to general formula (II) is selected from:

- glyoxal,
- glyoxylic acid,
- chloral. bromal, fluoral,
- dichloroacetone,
- trifluoroacetone,
- tritluoroacetophenone,
- trifluoroacetylacetone,
- methyl pyruvate and ethyl pyruvate.

**8.** A process according to one of Claims 1 to 7, characterised in that the quaternary ammonium hydroxide used corresponds to formula (III):

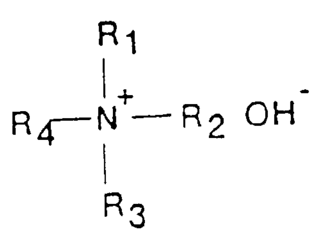

(III)

in which formula (III), the $R_1$, $R_2$, $R_3$ and $R_4$ radicals which are the same or different represent:

- a straight or branched, optionally substituted, alkyl radical with 1 to 20 carbon atoms, preferably 1 to 12 carbon atoms, preferably methyl, ethyl, butyl,
- an optionally substituted aryl radical with 6 to 12 carbon atoms, preferably 6 to 10 carbon atoms, preferably phenyl,
- an optionally substituted aralkyl radical with 7 to 14 carbon atoms, preferably 7 to 10 carbon atoms, preferably benzyl,
- two of said $R_1$ to $R_4$ radicals being capable of together forming a straight or branched alkylene radical with 3 to 6 carbon atoms.

**9.** A process according to one of Claims 1 to 8, characterised in that the quaternary ammonium hydroxide used is a tetralkylammonium hydroxide or a trialkylbenylammonium hydroxide, whose alkyl radicals which can be the same or different represent a straight or branched alkyl chain with 1 to 12 carbon atoms, preferably 1 to 6 carbon atoms.

**10.** A process according to one of Claims 1 to 9, characterised in that the quaternary ammonium hydroxide used is tetramethylammonium hydroxide, tetraethylammonium hydroxide, tetrabutylammonium hydroxide, trimethylbenzylammonium hydroxide.

**11.** A process according to one of Claims 1 to 10, characterised in that the aqueous solution of the carbonyl compound of formula (II) has a concentration varying between 15 and 100% by weight.

**12.** A process according to one of Claims 1 to 11, characterised in that the molar ratio between the hydroxylated aromatic compound of formula (I) and the carbonyl compound of formula (II) varies bctween 0.3 and 4.0 and is preferably selected between 0.5 and 2.0.

**13.** A process according to one of Claims 1 to 12, characterised in that the aqueous solution of quaternary ammonium hydroxide has a concentration varying between 10 and 50% by weight.

**14.** A process according to one of Claims 1 to 13, characterised in that the amount of quaternary ammonium hydroxide varies between 80 and 120% of the stoichiometric quantity needed to salify all the salifiable groups of the hydroxylated aromatic compound of formula (I) and of the carbonyl compound of formula (II).

**15.** A process according to one of Claims 1 to 14, characterised in that the concentration of the hydroxylated aromatic compound of formula (I) is preferably between 0.5 and 1.5 moles/litre, and, more particularly, approximately 1 mole/litre.

**16.** A process according to one of Claims 1 to 15, characterised in that the reaction temperature varies between 20°C and 60°C, preferably between 30°C and 40°C.

**17.** A process according to Claim 1, characterised in that the 3-methoxy-p-hydroxymandelic acid is prepared by condensing guaiacol and glyoxylic acid in water and in the presence of quaternary ammonium hydroxide.

**18.** Use of the product obtained in accordance with the preparation process described in one of Claims 1 to 17 as an intermediate for the production of hydroxy aryl acetic acids, hydroxy aryl glyoxylic acids or hydroxy aromatic aldehydes.

**19.** Use of the p-hydroxymandelic acid and 3-methoxy-p-hydroxymandelic, 3-ethoxy p-hydroxymandelic or 3-isopropoxy p-hydroxymandelic acids obtained in accordance with the preparation process described in one of Claims 1 to 17 for the production of 4-hydroxybenzaldehyde and vanillin, and the like, by oxidation of said acids.